# EUROPEAN PATENT APPLICATION

(11) **EP 4 449 974 A1**
(43) Date of publication of application: **23.10.2024**
(21) Application number: 23814736.7
(22) Date of filing: 29.03.2023
(51) Int. Cl.: A61B 1/303, A61B 1/015

(54) **HYSTEROSCOPE OPERATING SYSTEM**

(30) Priority: 30.05.2022 CN 202210595939
(71) Applicant: Shanghai Yodo Medical Technologies AG Co., Ltd, Shanghai 201318 (CN)
(72) Inventor: YANG, Xiang, Shanghai 201318 (CN); HAO, Jinzheng, Shanghai 201318 (CN); WANG, Chenfeng, Shanghai 201318 (CN)
(74) Representative: Ridderbusch, Oliver
(86) International application number: PCT/CN2023/084589
(87) International publication number: WO 2023/231539

(57) **Abstract**

The present invention discloses a hysteroscope operating system, including: an electronic hysteroscope which includes a hysteroscope body and a sheath tube mounted at a far end of the hysteroscope body and is further provided with an instrument channel and a water return channel which penetrate through the sheath tube and the hysteroscope body, wherein a far end of the sheath tube is provided with a water inlet which communicates with the instrument channel and a water return port which communicates with the water return channel; an intrauterine perfusion system which includes a perfusion body, a water inlet assembly and a water return assembly, wherein a proximal end of the water inlet assembly is connected to the perfusion main body while a far end of the same is connected to the instrument channel, and a proximal end of the water return assembly is connected to the perfusion main body while a far end of the same is connected to the water return channel; and a control mechanism which includes a first pressure sensor and a second pressure sensor, wherein the first pressure sensor is mounted at the water inlet, and the second pressure sensor is mounted at the water return port. The first pressure sensor and the second pressure sensor are arranged at the far end of the sheath tube, so that the actual pressure in a uterine cavity can be directly detected during use, and an operator can conveniently and accurately know the real pressure in the uterine cavity.

## Description

### FIELD OF THE INVENTION

The present invention relates to the field of medical treatment, and further relates to a hysteroscope operating system.

### BACKGROUND OF THE INVENTION

A hysteroscope is a surgical instrument which is often used in gynecological surgery. An operator can use the hysteroscope to observe the situation inside the uterus. The hysteroscope can also serve as a channel for surgical instruments to pass through in order to perform corresponding uterine surgical operations.

During using the hysteroscope to perform intrauterine examination or intrauterine surgery, it is usually necessary to use a perfusion device to perfuse uterine cavity for a uterine distension operation, thus increasing the volume of the uterine cavity and facilitate observation or surgical operation.

A fluid sensor of an existing intrauterine perfusion device is usually arranged at a proximal end of the intrauterine perfusion device, and it usually cannot accurately detect the actual pressure in the uterine cavity, so there is a high risk that the intrauterine pressure exceeds the maximum pressure that can be tolerated during use, or the perfusion pressure in the uterine cavity is insufficient during use, which affects the operator observation or the performance of intrauterine surgery.

It is to be noted that, in actual use, the presence of a drainage tube will further increase the distance between a distal end and the proximal end of the intrauterine perfusion device, which may further increase the difficulty in determining the actual intrauterine pressure.

### SUMMARY OF THE INVENTION

For technical problems above, an objective of the present invention is to provide a hysteroscope operating system. A first pressure sensor and a second pressure sensor are arranged at a far end of a sheath tube, so that the actual pressure in the uterine cavity can be directly detected during use, and an operator can conveniently and accurately know the real pressure in the uterine cavity.

In order to achieve the above object, the present invention provides a hysteroscope operating system, comprising:
an electronic hysteroscope comprising a hysteroscope body and a sheath tube mounted at a far end of the hysteroscope body, wherein the electronic hysteroscope is further provided with an instrument channel and a water return channel which penetrate through the sheath tube and the hysteroscope body, and a far end of the sheath tube is provided with a water inlet which communicates with the instrument channel, and a water return port which communicates with the water return channel;
an intrauterine perfusion system comprising a perfusion body, a water inlet assembly and a water return assembly, wherein a proximal end of the water inlet assembly is connected to the perfusion body while a far end of the same is connected to the instrument channel, and a proximal end of the water return assembly is connected to the perfusion body while a far end of the same is connected to the water return channel; and
a control mechanism comprising a first pressure sensor and a second pressure sensor, wherein the first pressure sensor is mounted at the water inlet and used for detecting the water flow pressure of the water inlet, and the second pressure sensor is mounted at the water return port and used for detecting the water flow pressure of the water return port.

In some preferred embodiments of the present invention, the control mechanism further includes a controller; the first pressure sensor and the second pressure sensor are respectively connected to the controller; and only when the pressure of the first pressure sensor and the pressure of the second pressure sensor are larger than a preset value, the water return assembly is controlled to be started.

In some preferred embodiments of the present invention, the control mechanism further comprises a controller which is in communication connection with the first pressure sensor and the second pressure sensor; in an injection stage, the controller controls the water inlet assembly and the water return assembly to inject water into the far end of the sheath tube at the same time; in a circulation stage, when the pressure of the first pressure sensor and the pressure of the second pressure sensor are both larger than a preset value, the water inlet assembly is controlled to inject water, and the water return assembly is controlled to return water; in a water return stage, the controller controls the water inlet assembly and the water return assembly to return water at the same time;

In some preferred embodiments of the present invention, the first pressure sensor is located on a far end face of the sheath tube, and the second pressure sensor is located on a far end side face of the sheath tube.

In some preferred embodiments of the present invention, the hysteroscope operating system further comprises a lens assembly mounted at the far end of the sheath tube, wherein the lens assembly comprises a lens set, a light sensing chip and a circuit board; the lens set is located on a light sensing path of the light sensing chip; the light sensing chip is electrically connected to the circuit board; and the first pressure sensor and the second pressure sensor are electrically connected to the circuit board.

In some preferred embodiments of the present invention, the circuit board comprises a first connecting plate and a second connecting plate; the first connecting plate is arranged in a radial direction of the sheath tube, the second connecting plate is arranged in a length extending direction of the sheath tube, and the first connecting plate is connected to the second connecting plate; and the first pressure sensor and the photosensitive chip are respectively electrically connected to the first connecting plate, and the second pressure sensor is electrically connected to the second connecting plate.

In some preferred embodiments of the present invention, the control mechanism further comprises a third pressure sensor and a fourth pressure sensor; the third pressure sensor is mounted in a middle area of a water flow path of the water inlet assembly; the fourth pressure sensor is mounted in a middle area of a water return path of the water return assembly, and the third pressure sensor and the fourth pressure sensor are in communication connection with the controller; and the controller is used for controlling the work of the perfusion body based on the detection data of the third pressure sensor and the fourth pressure sensor.

In some preferred embodiments of the present invention, the hysteroscope operating system further comprises a first T-joint and a second T-joint which are mounted on the hysteroscope body; the first T-joint is located on the water flow path of the water inlet assembly; the second T-joint is located on the water flow path of the water return assembly; the third pressure sensor is mounted on the first T-joint; and the fourth pressure sensor is mounted on the second T-joint.

In some preferred embodiments of the present invention, the hysteroscope body further comprises a control mainboard, the third pressure sensor and the fourth pressure sensor are respectively electrically connected to the control mainboard, and the control mainboard is electrically connected to the circuit board.

In some preferred embodiments of the present invention, the perfusion body comprises a sewage barrel and a clean barrel; the water inlet assembly comprises a water inlet pipe, a first control valve and a first branch pipe; a far end of the water inlet pipe communicates with the instrument channel while a proximal end of the same communicates with the first control valve; the first control valve communicates with the clean barrel; one end of the first branch pipe communicates with the first control valve while the other end of the same communicates with the sewage barrel;
the water return assembly comprises a water return pipe, a second control valve and a second branch pipe; a far end of the water return pipe communicates with the water return channel while a proximal end of the same communicates with the sewage barrel; and one end of the second branch pipe communicates with the second control valve while the other end of the same communicates with the clean barrel.

Compared with related technology, the hysteroscope operating system according to the present invention has at least one of the following beneficial effects:
1. According to the hysteroscope operating system provided by the present invention, the first pressure sensor and the second pressure sensor are arranged at the far end of the sheath tube, so that the actual pressure in the uterine cavity can be directly detected during use, and the operator can conveniently and accurately know the real pressure in the uterine cavity.
2. According to the hysteroscope operating system provided by the present invention, The first pressure sensor is arranged at the water inlet and can detect the real pressure of the water inlet; the second pressure sensor is arranged at the water outlet and can detect the real pressure of the water outlet; and the operator can be allowed to acquire the pressure of the water inlet and the pressure of the water outlet, and thus pressure control can be carried out more accurately.
3. According to the hysteroscope operating system provided by the present invention, a circuit board includes a first connecting plate and a second connecting plate; the first connecting plate is arranged in a radial direction of the sheath tube, the second connecting plate is arranged in a length extending direction of the sheath tube, and the first connecting plate is connected to the second connecting plate; the first pressure sensor and a photosensitive chip are electrically connected to the first connecting plate respectively, and the second pressure sensor is electrically connected to the second connecting plate, thus the first pressure sensor and the second pressure sensor can be mounted without additionally arranging the circuit board, and a mounting structure is simplified.
4. According to the hysteroscope operating system provided by the present invention, In the injection stage at the early stage of an operation, the controller controls the water inlet assembly and the water return assembly to inject water into the far end of the sheath tube at the same time, thus the speed of injecting water into the uterine cavity can be increased, and the liquid injection efficiency is improved. In the water return stage at the later stage of the operation, the controller controls the water inlet assembly and the water return assembly to return water at the same time, and thus the efficiency of discharging liquid in the uterine cavity can be improved.

### BRIEF DESCRIPTION OF THE DRAWINGS

The preferred embodiments will be described below in a clear and understandable manner with reference to the accompanying drawings, so as to further describe the characteristics, technical features, advantages and implementation methods of the present invention.
FIG. 1 is a three-dimensional structure schematic diagram of a hysteroscope operating system according to a preferred embodiment of the present invention;
FIG. 2 is a side structure schematic diagram of a hysteroscope of a hysteroscope operating system according to a preferred embodiment of the present invention;
FIG. 3 is a sectional view structure schematic diagram of a hysteroscope of a hysteroscope operating system according to a preferred embodiment of the present invention;
FIG. 4 is an enlarged diagram of a part a in FIG. 3;
FIG. 5 is an enlarged diagram of a part b in FIG. 3; and
FIG. 6 is a structure schematic diagram of a head end face of a sheath tube of a hysteroscope operating system according to a preferred embodiment of the present invention.

Mark number in figures:
Electronic hysteroscope 10, hysteroscope body 11, instrument channel 111, water return channel 112, control mainboard 113, sheath tube 12, lens assembly 13, lens set 131, light sensing chip 132, circuit board 133, first connecting plate 1331, second connecting plate 1332, illumination element 134, first T-joint 141, second T-joint 142, connecting plug 15, intrauterine perfusion system 20, perfusion body 21, sewage barrel 211, clean barrel 212, water inlet assembly 22, water inlet pipe 221, first control valve 222, first branch pipe 223, water return assembly 23, water return pipe 231, second control valve 232, second branch pipe 233, control mechanism 30, first pressure sensor 31, second pressure sensor 32, third pressure sensor 34, and fourth pressure sensor 35.

### DETAILED DESCRIPTION OF SOME EMBODIMENTS

In order to more clearly illustrate embodiments of the present invention or technical solutions in the prior art, the detailed implementations of the present invention will be described below by reference to the accompanying drawings. Apparently, the accompanying drawings in the following description show merely some embodiments of the present invention, and a person of ordinary skill in the art may still derive other drawings from these accompanying drawings without creative efforts, and derive other implementations.

For the sake of brevity, each drawing only schematically represents the parts related to the present invention, but they do not represent their actual structures as products. Further, in order to make the drawings concise and easy to understand, for parts in some drawings that have the same structure or function, only one of the parts is schematically depicted, or only marked. "One" herein means a case of not only "only this one", but also "more than one".

It should also be further understood that the term "and/or" as used in the description of the present application and the accompanying claims refers to any combination of one or more of the associated listed items and all possible combinations, and includes these combinations.

In the description of the present invention, it should be noted that, unless otherwise definitely specified and limited, the terms "mounted", "connected with each other", and "connected to/with" need to be broadly understood, for example, connection may be fixed connection, or detachable connection or integrated connection; or may be mechanical connection or electric connection; or may be direct connection, or indirect connection via an intermediation, or communication of inner parts of two elements. A person of ordinary skill in the art can understand the specific meaning of the above terms in the present invention in accordance with specific conditions.

Furthermore, in the description of the present application, the terms "first" and "second" are used for a distinguishing description only and are not to be construed as indicating or implying relative importance.

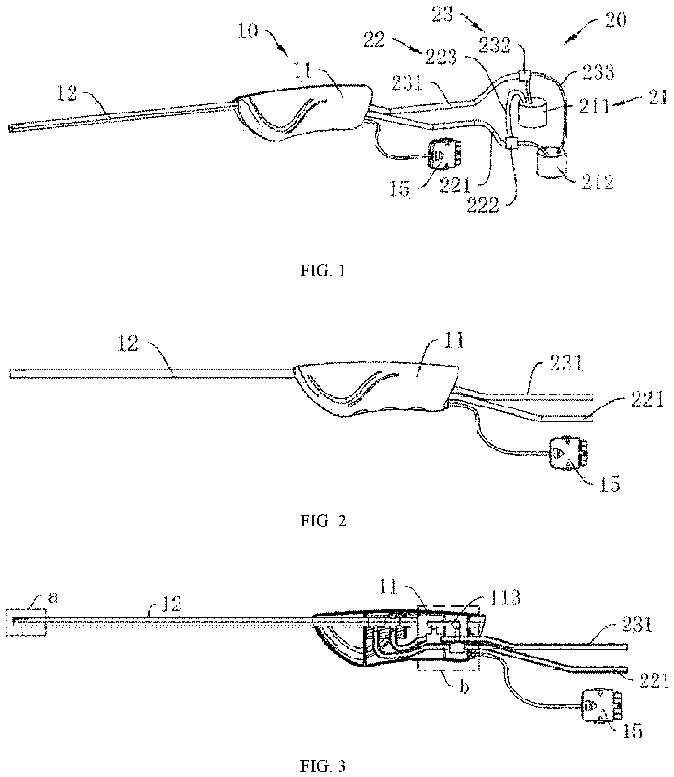

As shown in FIG. 1 to FIG. 6, a hysteroscope operating system according to a preferred embodiment of the present invention includes an electronic hysteroscope 10, an intrauterine perfusion system 20 and a control mechanism 30. The electronic hysteroscope 10 includes a hysteroscope body 11 and a sheath tube 12 mounted at a far end of the hysteroscope body 11; the electronic hysteroscope 10 is further provided with an instrument channel 111 and a water return channel 112 which penetrate through the sheath tube 12 and the hysteroscope body 11; and a far end of the sheath tube 12 is provided with a water inlet which communicates with the instrument channel 111, and a water return port which communicates with the water return channel 112. The intrauterine perfusion system 20 includes a perfusion body 21, a water inlet assembly 22 and a water return assembly 23; a proximal end of the water inlet assembly 22 is connected to the perfusion body 21 while a far end of the same is connected to the instrument channel 111; a proximal end of the water return assembly 23 is connected to the perfusion body 21 while a far end of the same is connected to the water return channel 112. The control mechanism 30 includes a first pressure sensor 31 and a second pressure sensor 32; the first pressure sensor 31 is mounted at the water inlet and used for detecting the water flow pressure of the water inlet; and the second pressure sensor 32 is mounted at the water return port and used for detecting the water flow pressure of the water return port.

The first pressure sensor 31 and the second pressure sensor 32 are arranged at the far end of the sheath tube 12, so that the actual pressure in a uterine cavity can be directly detected during use, and an operator can conveniently and accurately know the real pressure in the uterine cavity. The first pressure sensor 31 is arranged at the water inlet and can detect the real pressure of the water inlet; the second pressure sensor 32 is arranged at the water outlet and can detect the real pressure of the water outlet; and the operator can be allowed to acquire the pressure of the water inlet and the pressure of the water outlet, and thus pressure control can be carried out more accurately.

The control mechanism 30 further includes a controller (not shown in the figure); the first pressure sensor 31 and the second pressure sensor 32 are respectively connected to the controller; and only when the pressure of the first pressure sensor 31 and the pressure of the second pressure sensor 32 are larger than a preset value, the water return assembly 23 is controlled to be started.

In an existing intrauterine perfusion system, the water inlet assembly 22 of the intrauterine perfusion system 20 can be controlled to feed water for a certain time, and then the water return assembly 23 is opened to return water; when an instrument is subjected to water leakage and the like, the water fed through the water inlet assembly 22 may not completely enter the uterine cavity, consequently, liquid in the uterine cavity cannot meet the requirement, and at the moment, if the water return assembly 23 is pneumatically operated to return water, the pressure in the uterine cavity may not reach the preset pressure all the time, and surgical operation may be carried out in an incompletely expanded uterine cavity. In this preferred embodiment, only when the pressure of the first pressure sensor 31 and the pressure of the second pressure sensor 32 are both larger than the preset pressure, the water return assembly 23 is controlled to be started for water return operation, and the water return operation can be carried out only after the pressure in the uterine cavity reaches the preset value. On the other hand, after the first pressure sensor 31 and the second pressure sensor 32 both reach the preset value, the water return assembly 23 is started to for the water return operation, then the pressure in the uterine cavity can quickly reach the preset value, and thus the uterine expansion efficiency is improved.

Preferably, the water inlet of the instrument channel 111 is located in the far end face of the sheath tube 12, the water return port of the water return channel 112 is located in a far end side face of the sheath tube 12, and the water inlet and the water return port are located in different planes, so that a flowing path of injected liquid in the uterine cavity can be improved, and the flushing effect can be improved. Correspondingly, the first pressure sensor 31 is located on the far end face of the sheath tube 12, and the second pressure sensor 32 is located on the far end side face of the sheath tube 12.

The hysteroscope operating system further includes a lens assembly 13 mounted at the far end of the sheath tube 12. The lens assembly 13 includes a lens set 131, a light sensing chip 132 and a circuit board 133; the lens set 131 is located on a light sensing path of the light sensing chip 132; the light sensing chip 132 is electrically connected to the circuit board 133; and the first pressure sensor 31 and the second pressure sensor 32 are respectively electrically connected to the circuit board 133.

The first pressure sensor 31, the second pressure sensor 32 and the light sensing chip 132 share the circuit board 133, so a new circuit board does not need to be additionally arranged, and moreover, the use efficiency of the circuit board 133 can be improved.

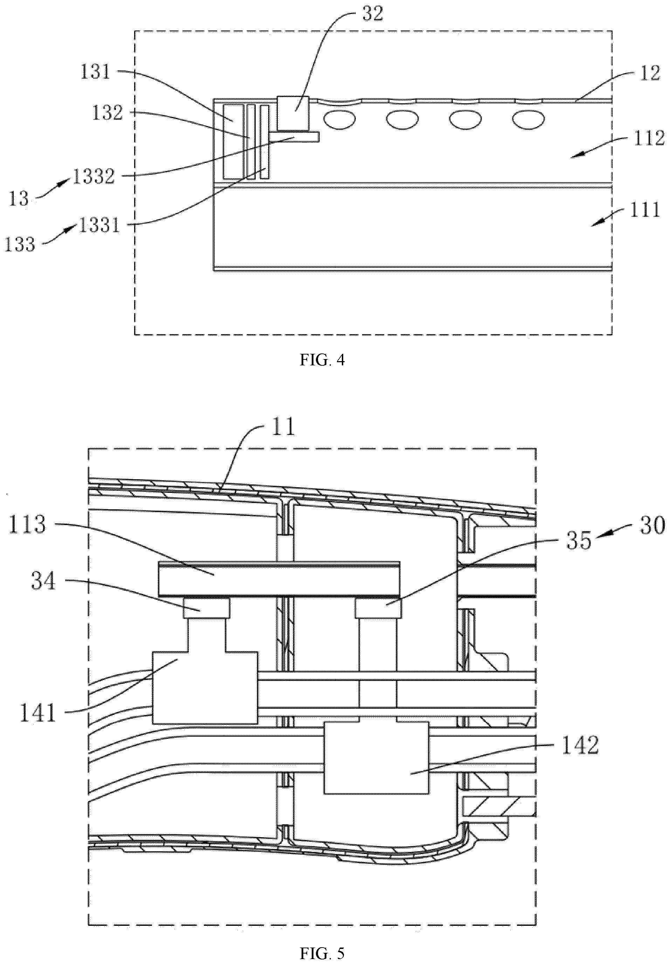

As shown in FIG. 4, the electronic hysteroscope 10 further includes an illumination element 134 arranged on the far end face of the sheath tube 12, and the illumination element 134 is arranged on one side of the lens assembly 13 and can provide illumination during use. Preferably, the illumination element 134 is an LED lamp.

As shown in FIG. 5, the circuit board 133 includes a first connecting plate 1331 and a second connecting plate 1332. The first connecting plate 1331 is arranged in a radial direction of the sheath tube 12, the second connecting plate 1332 is arranged in a length extending direction of the sheath tube 12, and the first connecting plate 1331 is connected to the second connecting plate 1332. The first pressure sensor 31 and the photosensitive chip 132 are respectively electrically connected to the first connecting plate 1331, and the second pressure sensor 32 is electrically connected to the second connecting plate 1332.

Preferably, the first connecting plate 1331 and the second connecting plate 1332 are interconnected to form a T-shaped structure. In a deformation embodiment, the first connecting plate 1331 and the second connecting plate 1332 are interconnected to form an L-shaped structure. The circuit board 133 is arranged to be provided with the first connecting plate 1331 and the second connecting plate 1332 which have a certain cross angle, thus the first pressure sensor 31, the second pressure sensor 32 and the photosensitive chip 132 can be connected through one circuit board at the same time, improving the space utilization efficiency.

As shown in FIG. 5, the control mechanism 30 further includes a third pressure sensor 34 and a fourth pressure sensor 35. The third pressure sensor 34 is mounted in a middle area of a water flow path of the water inlet assembly 22; the fourth pressure sensor 35 is mounted in a middle area of a water return path of the water return assembly 23, the third pressure sensor 34 and the fourth pressure sensor 35 are respectively in communication connection with the controller; and the controller is used for controlling the work of the perfusion body 21 based on the detection data of the third pressure sensor 34 and the fourth pressure sensor 35.

The third pressure sensor 34 and the fourth pressure sensor 35 are arranged, so that the pressure data of a plurality of points on the water inlet path and the water return path can be acquired, which facilitates the detection of leakage faults. For example, during normal work, the pressure of the middle area of the water inlet assembly 22 is approximately equal to the pressure of the middle area of the water return assembly 23; if the pressure of the third pressure sensor 34 is larger than the pressure of the fourth pressure sensor 35, it indicates that the water return port in the far end of the sheath tube 12 is blocked, and if water return cannot be stopped in time, damage may be caused to the uterine cavity.

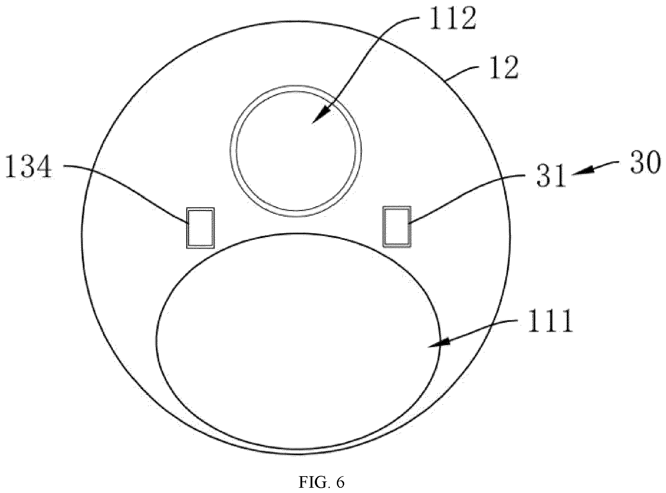

As shown in FIG. 6, the hysteroscope operating system further includes a first T joint 141 and a second T-joint 142 which are mounted on the hysteroscope body 11; the first T-joint 141 is located on the water flow path of the water inlet assembly 22; the second T-joint 142 is located on the water flow path of the water return assembly 23; the third pressure sensor 34 is mounted on the first T-joint 141; and the fourth pressure sensor 35 is mounted on the second T-joint 142.

Because the first T-joint 141 and the second T-joint 142 are arranged, the third pressure sensor 34 and the fourth pressure sensor 35 can be arranged without influencing normal water inlet and water return; and therefore, the mounting structure of the third pressure sensor 34 and the fourth pressure sensor 35 is simplified.

The hysteroscope body 11 further includes a control mainboard 113, the third pressure sensor 34 and the fourth pressure sensor 35 are respectively electrically connected to the control mainboard 113, and the control mainboard 113 is electrically connected to the circuit board 133. The third pressure sensor 34 and the fourth pressure sensor 35 are respectively electrically connected to the control mainboard 113, and power can be supplied to the third pressure sensor 34 and the fourth pressure sensor 35 without additionally arranging a new circuit board, so that the mounting structure is simplified.

In a deformation embodiment, in an injection stage, the controller controls the water inlet assembly 22 and the water return assembly 23 to inject water into the far end of the sheath tube 12 at the same time; in a circulation stage, when the pressure of the first pressure sensor 31 and the pressure of the second pressure sensor 32 are both larger than the preset value, the water inlet assembly 22 is controlled to inject water, and the water return assembly 23 is controlled to return water; and in a water return stage, the controller controls the water inlet assembly 22 and the water return assembly 23 to return water at the same time.

In the injection stage at the early stage of an operation, the controller controls the water inlet assembly 22 and the water return assembly 23 to inject water into the far end of the sheath tube 12 at the same time, thus the speed of injecting water into the uterine cavity can be increased, and the liquid injection efficiency is improved. In the water return stage at the later stage of the operation, the controller controls the water inlet assembly 22 and the water return assembly 23 to return water at the same time, and thus the efficiency of discharging liquid in the uterine cavity can be improved.

As shown in FIG. 1, the perfusion body 21 includes a sewage barrel 211 and a clean barrel 212. The water inlet assembly 22 includes a water inlet pipe 221, a first control valve 222 and a first branch pipe 223; a far end of the water inlet pipe 221 communicates with the instrument channel 111 while a proximal end of the same communicates with the first control valve 222; the first control valve 222 communicates with the clean barrel 212; and one end of the first branch pipe 223 communicates with the first control valve 222 while the other end of the same communicates with the sewage barrel 211. The water return assembly 23 includes a water return pipe 231, a second control valve 232 and a second branch pipe 233; a far end of the water return pipe 231 communicates with the water return channel 112 while a proximal end of the same communicates with the sewage barrel 211; and one end of the second branch pipe 233 communicates with the second control valve 232 while the other end of the same communicates with the clean barrel 212.

In the injection stage at the early stage of an operation, the controller controls the working state of the first control valve 222, the second control valve 232 and the perfusion body 21, and liquid in the clean barrel 212 enters the instrument channel and the water return channel through the water inlet pipe 221 and the second branch pipe 233 respectively, so that water injection operation is implemented on the uterine cavity; in the water return stage at the later stage of the operation, the controller controls the working states of the first control valve 222, the second control valve 232 and the perfusion body 21, liquid in the instrument channel enters the sewage barrel 211 through the first branch pipe 223, and liquid in the water return pipe 231 enters the sewage barrel 211. In the circulation stage, the controller controls the working states of the first control valve 222, the second control valve 232 and the perfusion body 21, liquid in the clean barrel 212 enters the instrument channel through the water inlet pipe 221, and liquid in the uterine cavity enters the sewage barrel 211 through the water return pipe 231.

As shown in FIG. 1, FIG. 2 and FIG. 3, the electronic hysteroscope 10 further includes a connecting plug 15 electrically connected to the hysteroscope body 11, and the connecting plug 15 is connected to external devices to implement the functions of data transmission and electrification.

Preferably, a suction pump is arranged in the sewage barrel 211 of the perfusion body 21 and used for sucking liquid from the water return channel 112 into the sewage barrel 211; a pressure pump is arranged in the clean barrel 212 and used for driving liquid in the clean barrel 211 to enter the instrument channel 111. In a deformation embodiment, the suction pump and the pressure pump can also be arranged on the outer side of the sewage barrel 211 and the outer side of the clean barrel 212.

It should be noted that the above embodiments may be freely combined as needed. The above description is only preferred embodiments of the present invention, and it should be pointed out that those of ordinary skill in the art may also make several improvements and modifications without departing from the principles of the present invention, which should be considered as the protection scope of the present invention.

## Claims

1. A hysteroscope operating system, comprising:
an electronic hysteroscope comprising a hysteroscope body and a sheath tube mounted at a far end of the hysteroscope body, wherein the electronic hysteroscope is further provided with an instrument channel and a water return channel which penetrate through the sheath tube and the hysteroscope body, and a far end of the sheath tube is provided with a water inlet which communicates with the instrument channel, and a water return port which communicates with the water return channel;
an intrauterine perfusion system comprising a perfusion body, a water inlet assembly and a water return assembly, wherein a proximal end of the water inlet assembly is connected to the perfusion body while a far end of the same is connected to the instrument channel, and a proximal end of the water return assembly is connected to the perfusion body while a far end of the same is connected to the water return channel; and
a control mechanism comprising a first pressure sensor and a second pressure sensor, wherein the first pressure sensor is mounted at the water inlet and used for detecting the water flow pressure of the water inlet, and the second pressure sensor is mounted at the water return port and used for detecting the water flow pressure of the water return port;
the control mechanism further comprises a controller which is in communication connection with the first pressure sensor and the second pressure sensor; in an injection stage, the controller controls the water inlet assembly and the water return assembly to inject water into the far end of the sheath tube at the same time; in a circulation stage, when the pressure of the first pressure sensor and the pressure of the second pressure sensor are both larger than a preset value, the water inlet assembly is controlled to inject water, and the water return assembly is controlled to return water; in a water return stage, the controller controls the water inlet assembly and the water return assembly to return water at the same time;
the perfusion body comprises a sewage barrel and a clean barrel; the water inlet assembly comprises a water inlet pipe, a first control valve and a first branch pipe; a far end of the water inlet pipe communicates with the instrument channel while a proximal end of the same communicates with the first control valve; the first control valve communicates with the clean barrel; one end of the first branch pipe communicates with the first control valve while the other end of the same communicates with the sewage barrel; the water return assembly comprises a water return pipe, a second control valve and a second branch pipe; a far end of the water return pipe communicates with the water return channel while a proximal end of the same communicates with the sewage barrel; and one end of the second branch pipe communicates with the second control valve while the other end of the same communicates with the clean barrel.

2. The hysteroscope operating system according to claim 1, wherein the first pressure sensor is located on a far end face of the sheath tube, and the second pressure sensor is located on a far end side face of the sheath tube.

3. The hysteroscope operating system according to claim 2, further comprising a lens assembly mounted at the far end of the sheath tube, wherein the lens assembly comprises a lens set, a light sensing chip and a circuit board; the lens set is located on a light sensing path of the light sensing chip; the light sensing chip is electrically connected to the circuit board; and the first pressure sensor and the second pressure sensor are electrically connected to the circuit board.

4. The hysteroscope operating system according to claim 3, wherein the circuit board comprises a first connecting plate and a second connecting plate; the first connecting plate is arranged in a radial direction of the sheath tube, the second connecting plate is arranged in a length extending direction of the sheath tube, and the first connecting plate is connected to the second connecting plate; and the first pressure sensor and the photosensitive chip are respectively electrically connected to the first connecting plate, and the second pressure sensor is electrically connected to the second connecting plate.

5. The hysteroscope operating system according to claim 3, wherein the control mechanism further comprises a third pressure sensor and a fourth pressure sensor; the third pressure sensor is mounted in a middle area of a water flow path of the water inlet assembly; the fourth pressure sensor is mounted in a middle area of a water return path of the water return assembly, and the third pressure sensor and the fourth pressure sensor are in communication connection with the controller; and the controller is used for controlling the work of the perfusion body based on the detection data of the third pressure sensor and the fourth pressure sensor.

6. The hysteroscope operating system according to claim 5, wherein the hysteroscope operating system further comprises a first T-joint and a second T-joint which are mounted on the hysteroscope body; the first T-joint is located on the water flow path of the water inlet assembly; the second T-joint is located on the water flow path of the water return assembly; the third pressure sensor is mounted on the first T-joint; and the fourth pressure sensor is mounted on the second T-joint.

7. The hysteroscope operating system according to claim 5, wherein the hysteroscope body further comprises a control mainboard, the third pressure sensor and the fourth pressure sensor are respectively electrically connected to the control mainboard, and the control mainboard is electrically connected to the circuit board.
